# EUROPÄISCHE PATENTSCHRIFT

(11) **EP 0 584 484 B1**
(45) Veröffentlichungstag und Bekanntmachung des Hinweises auf die Patenterteilung: **13.10.1999**
(21) Anmeldenummer: 93109964.2
(22) Anmeldetag: 22.06.1993
(51) Int. Cl.: A61L 2/00, A61L 2/04

(54) **Verfahren und Einrichtung zum Desinfizieren von Knochentransplantaten, insbesondere von humanen Spongiosa-Transplantaten**
Method and apparatus for disinfecting bone transplants, especially human spongiosa transplants
Procédé et appareil pour la désinfection de transplants osseux, en particulier de transplants de tissus spongieux humain

(30) Priorität: 21.08.1992 DE 4227830
(43) Veröffentlichungstag der Anmeldung: 02.03.1994
(73) Patentinhaber: Tulaszewski, Olaf, D-35037 Marburg (DE)
(72) Erfinder: Knaepler, Harald, Priv. Dozent Dr., D-3557 Beltershausen (DE); von Garrel, Thomas, Dr., D-3550 Marburg (DE)
(74) Vertreter: TER MEER STEINMEISTER & PARTNER GbR

(56) Entgegenhaltungen:
- Keine einschlägigen Dokumente gefunden

## Beschreibung

Die Erfindung bezieht sich auf ein Verfahren und auf eine Einrichtung zum Desinfizieren von Knochentransplanlaten, insbesondere von humanen Spongiosa-Transplantaten, gemäß den Patentansprüchen 1 und 7.

Aufgrund von Erhebungen von Fachleuten werden gegenwärtig pro Jahr etwa 15.000 bis 25.000 allogene Transplantationen in Kliniken der Bundesrepublik Deutschland durchgeführt. Die Gefahr der Übertragung bakterieller und viraler Infektionserreger bei der Verwendung allogener Knochentransplantate stellt dabei ein nicht zu unterschätzendes medizinisches und juristisches Problem dar. Nach dem Bekanntwerden der ersten nachgewiesenen HIV-Übertragung durch ein allogenes, kryokonserviertes Knochentransplantat wurden bereits frühzeitig für die Bundesrepublik Deutschland verbindliche Richtlinien zum Führen von Knochenbanken veröffentlicht. Dabei führt besonders der darin geforderte HIV-Wiederholungstest des Spenders nach drei Monaten zu einem erheblichen personellen und organisatorischen Mehraufwand, ohne eine vollständige Sicherheit zu gewährleisten. Man war daher gezwungen, die Suche nach einem geeigneten Sterilisations- oder Desinfektionsverfahren aufzunehmen. Neben der Wärmebehandlung bieten sich dabei chemische und strahlensterilisierende Verfahren an. Das Hauptproblem bei der Verwendung chemischer Desinfektionsmittel ist, neben der möglichen Toxizität und Mutagenität, die zumeist ungenügende Diffusion des Agens durch den Knochen. Bei der Anwendung ionisierender Strahlen zur Sterilisation treten erhebliche logistische Probleme auf, da die benötigte erregerinaktivierende Strahlendosis nur in industriellen Anlagen erreichbar ist.

Die Autoklavierung zur Transplantatsterilisation konnte in dem in Rede stehenden Bereich keinen festen Platz einnehmen, da durch sie Schädigungen der biomechanischen Eigenschaften auftreten können und das Autoklavieren eine zusätzliche Zerstörung der Proteinstrukturen bewirkt, wodurch das biologische Einbauverhalten negativ beeinflußt wird.

Eine Alternative zur Sterilisation durch Autoklavierung (im Temperaturbereich oberhalb von 100°C) stellt die schonende Desinfektion durch eine moderate Erwärmung auf Temperaturen von unter 100°C dar. Aufgrund seiner Thermolabilität läßt sich eine Inaktivierung des HIV schon bei Temperaturen über 60°C erzielen, wobei sich andererseits bei einer Transplantaterwärmung auf 80°C außerdem auch die wesentlich häufiger vorkommenden vegetativen Kontaminationskeime wie Staphylokokken und Streptokokken sicher abtöten lassen.

Insbesondere Spongiosamaterial, das gegenüber Kortikalistransplantaten eine deutliche Überlegenheit aufweist, wurde zu vielen Test herangezogen, um die Brauchbarkeit des thermischen Desinfektionsverfahrens nachzuweisen.

So ist bereits aus der DE 40 37 806 A1 ein Verfahren zur Desinfektion insbesondere von Knochentransplantaten durch thermische Behandlung in einem Inkubator bekannt. Um lange Aufheizzeiten und örtliche Überhitzungen zu vermeiden, wird außer einer thermostatgesteuerten Erwärmung des Inkubators in einem Wasserbad auch noch eine Hochfrequenzheizung eingesetzt, die In ihrer Wirkung direkt auf das Transplantat gerichtet wird. Beide Wärmequellen, die von einer zentralen Steuereinrichtung in Abhängigkeit von Temperatursensoren im jeweiligen Wirkungsbereich der Wärmequellen angesteuert werden, werden alternierend so geschaltet, daß eine vorgegebene Temperaturdifferenz im Transplantat nicht überschritten werden kann. Die Erwärmung der organischen Substanz geht auf diese Weise relativ rasch vor sich, ohne daß unzulässige Temperaturen und ein hoher momentaner Temperaturgradient zu befürchten wären. Auf diese Weise bleibt die biologische Wertigkeit und mechanische Festigkeit des Transplantats erhalten.

Das oben beschriebene Verfahren ist relativ aufwendig, da, wie erwähnt, Temperatursensoren im jeweiligen Wirkungsbereich der Wärmequellen positioniert werden müssen. Bei der DE 40 37 806 A1 wird dazu einer der Temperatursensoren im Zentrum des organischen Materials angeordnet. Es muß daher ein entsprechender Kanal in das Material eingebracht werden, wodurch sich das Risiko einer Kontamination erhöht.

Die entsprechende Behandlung des zu überprüfenden Transplantats läßt sich im allgemeinen nicht in einem Operationssaal durchführen, so daß das Transplantat auch über längere Wege zumindest im Klinikbereich zu transportieren ist, wodurch das Kontaminationsrisiko nochmals vergrößert wird.

Durch den Einsatz einer Hochfrequenzheizung oder Induktionsheizung als zweite Heizeinrichtung verteuert sich darüber hinaus die entsprechende Einrichtung zur Durchführung des Verfahrens erheblich, so daß seine Chancen, in großem Umfang in Kliniken ausgeführt zu werden, nur gering sind.

Der Erfindung liegt die Aufgabe zugrunde, ein Verfahren zum Desinfizieren von Knochentransplantaten zu schaffen, insbesondere von humanen Spongiosa-Transplantaten, das sich schnell und kostengünstig sowie unmittelbar im OP-Bereich ausführen läßt, und zwar ohne weitere Bearbeitung des Transplantats, um das Kontaminationsrisiko noch weiter zu verringern, Ziel der Erfindung ist es darüber hinaus, eine zur Durchführung dieses Verfahrens geeignete Einrichtung anzugeben.

Die verfahrensseitige Lösung der gestellten Aufgabe findet sich im Patentanspruch 1, während die vorrichtungsseitige Lösung der gestellten Aufgabe im Patentanspruch 7 angegeben ist. Vorteilhafte Ausgestaltungen der Erfindung sind den jeweils nachgeordneten Unteransprüchen zu entnehmen.

Ein erfindungsgemäßes Verfahren zum Desinfizieren von Knochentransplantaten, insbesondere von humanen Spongiosa-Transplantaten, zeichnet sich durch folgende Schritte aus:
a) Bereitstellen und Auswerten der Größe des Knochentransplantats,
b) Einlegen des Knochentransplantats in ein Gefäß mit vorbestimmten Fassungsvermögen,
c) Auffüllen des Gefäßes mit einer sterilen Flüssigkeit bis zu einer für alle Knochentransplantatgrößen vorgesehenen Gefäßmarkierung oberhalb des Knochentransplantats,
d) Verschließen des Gefäßes mit einem einen selbstabdichtenden Durchstoßbereich aufweisenden Deckel,
e) Einsetzen des verschlossenen Gefäßes in ein Heizgerät und Erhitzen des Gefäßes über eine konstante erste Zeitspanne auf eine Endtemperatur sowie Halten dieser Endtemperatur über eine in fester Beziehung zu der ausgemessenen Größe des Knochentransplantats stehenden zweiten Zeitspanne,
f) Abkühlen des Gefäßes auf Raumtemperatur,
g) Durchstoßen des Durchstoßbereichs des Deckels sowie eines Durchstoßbereichs eines anderen Deckels eines Auffangbehälters mittels eines Transfer-Sets zur Ableitung der Flüssigkeit aus dem Gefäß in den Auffangbehälter, und
h) Herausnehmen des Transfer-Sets aus dem Gefäßdeckel sowie Einbringen des Gefäßes in einen Kühlraum zwecks Einfrieren des Knochentransplantats.

Das oben beschriebene Verfahren kann durchgehend im OP-Bereich ausgeführt werden, so daß praktisch keine Gefahr mehr besteht, daß das Transplantat während des Desinfektionsverfahrens durch Krankheitserreger kontaminiert wird. Die größte Gefahrenquelle für eine derartige Kontamination, nämlich der Transport aus dem OP-Bereich heraus in ein geeignetes Labor, ggf. außerhalb der Klinik, ist somit beseitigt.

Nach Einfrieren des Transplantats im Kühlraum kann aus dem genannten Auffangbehälter Flüssigkeit wieder entnommen werden, um diese auf evtl. noch vorhandene Krankheitserreger hin zu überprüfen.

Das Verfahren nach der Erfindung eignet sich in ausgezeichneter Weise für den Routine-Transplantationsbetrieb in einem Klinikum, da es keiner besonderen Weiterbehandlung des Transplantats bedarf. Insbesondere ist es nicht mehr erforderlich, Temperatursensoren mit dem Transplantat zu verbinden, sondern es braucht lediglich zur Erhitzung in das Gefäß eingelegt zu werden. Nach Auffüllen des Gefäßes mit steriler Flüssigkeit, z. B. mit sterilem Wasser, erfolgt dann die Wärmebehandlung des Transplantats vollautomatisch, nachdem zuvor die wichtigste geometrische Abmessung des Transplantats in die Heizeinrichtung zu deren Steuerung eingegeben worden ist.

Besonders einfach ist das Verfahren deswegen, da für jede Transplantatgröße das Gefäß bis zu einer für alle Knochentransplantatgrößen vorgesehenen Gefäßmarkierung mit steriler Flüssigkeit aufgefüllt werden kann. Die Menge der zu verwenden Flüssigkeit braucht also nicht auf die Knochentransplantatgröße abgestimmt zu werden. Dies erleichtert den Betrieb wesentlich.

Sodann wird in einer ersten Zeitspanne, die für alle Knochentransplantatgrößen nur einen einzigen Wert aufweist, die sterile Flüssigkeit auf eine vorbestimmte Temperatur erhitzt. Nach Ablauf dieser ersten Zeitspanne weist somit die sterile Flüssigkeit, unabhängig von der Größe des Transplantats, wenigstens annähernd die gleiche Temperatur auf. Jedenfalls wird in allen Fällen eine Temperatur erreicht, die zur Desinfektion des Transplantats bzw. zur Abtötung der eingangs genannten Krankheitserreger völlig ausreicht.

Vorzugsweise ist die erste Zeitspanne in Übereinstimmung mit der Heizleistung der Heizeinrichtung so gewählt, daß nach Ablauf der ersten Zeitspanne die Flüssigkeit innerhalb des Gefäßes auf einer Temperatur von etwa 80°C zu liegen kommt. Die erste Zeitspanne kann z. B. 30 Minuten betragen. Sie kann aber auch kürzer sein, je nach Heizkapazität des Geräts.

Die Länge der sich daran anschließenden zweiten Zeitspanne wird in Abhängigkeit der ausgemessenen Größe des Knochentransplantats gewählt, wobei diese Größe zuvor in das Gerät eingegeben worden ist. Während dieser zweiten Zeitspanne wird die in der ersten Zeitspanne erreichte Endtemperatur aufrechterhalten, und zwar so lange, daß auch das Zentrum des Transplantats diese Endtemperatur annehmen kann. Danach bleibt die Endtemperatur weiterhin aufrechterhalten, und zwar so lange, bis die im Transplantat vorhandenen Krankheitserreger eliminiert worden sind. Diese sogenannte Abtötungszeit beträgt z. B. 10 Minuten. Die zweite Zeitspanne setzt sich somit aus der Abtötungszeit und derjenigen Zeit zusammen, die benötigt wird, damit auch das Zentrum des Transplantats die im ersten Schritt erreichte Endtemperatur annehmen kann. Die Zeiten für die jeweils zweite Zeitspanne lassen sich zuvor empirisch ermitteln und innerhalb eines Speichers des Geräts abspeichern, und zwar abhängig von den geometrischen Abmessungen des Knochentransplantats, so daß die zweiten Zeitspannen automatisch ausgewählt werden können, wenn die Abmessungen über eine Einstelleinrichtung in das Gerät eingeben werden. Handelt es sich um schichtförmige Transplantate, so wird als geometrische Abmessung die Schichtdicke eingegeben.

Um eine gleichmäßige Temperaturverteilung innerhalb der Flüssigkeit zu erzielen, kann diese durch geeignete Mittel verwirbelt werden, beispielsweise durch im Innern des Gefäßes befindliche Magnetteilchen, die durch ein äußeres Magnetdrehwerk bewegt werden. Dies wird weiter unten näher beschrieben. Diese Verwirbelung der Flüssigkeit kann auch bei deren Abkühlung erfolgen.

Vorzugsweise wird die Flüssigkeit beim Umfüllen vom Gefäß in den Auffangbehälter filtriert, um Rückstände im Gefäß zurückzubehalten. Hierzu ist eine entsprechende Filtereinrichtung am Auslaß des Gefäßes vorgesehen, worauf ebenfalls noch eingegangen wird.

Nach Ableitung der Flüssigkeit aus dem Gefäß wird dieses dann so positioniert, daß das Knochentransplantat getrennt von einer evtl. noch vorhandenen Restflüssigkeit sowie getrennt von den genannten Rückständen auf einem im Gefäßinnern vorhandenen Gitter zu liegen kommt. Auf diese Weise läßt sich das Knochentransplantat trocken und rückstandsfrei im Gefäß lagern, um dann zusammen mit dem Gefäß eingefroren zu werden.

Eine Einrichtung zur Durchführung des erfindungsgemäßen Verfahrens enthält eine Temperiervorrichtung mit einer Heizmulde und einer Kühlmulde; ein Gefäß zum Einlegen des Knochentransplantats mit vorbestimmtem Fassungsvermögen und einen einen selbstabdichtenden Durchstoßbereich aufweisenden Deckel; einen Auffangbehälter mit einem einen selbstabdichtenden Durchstoßbereich aufweisenden Deckel; ein Transfer-Set zur Ableitung der sterilen Flüssigkeit aus dem Gefäß in den Auffangbehälter; und eine Steuereinrichtung zum Steuern des Erhitzens bzw. des Abkühlens des Gefäßes.

Das Aufheizen und Abkühlen der Flüssigkeit bzw. des in ihr vorhandenen Knochentransplantats läßt sich dadurch an dicht nebeneinanderliegenden Plätzen bewerkstelligen, was der weiteren Vereinfachung des Verfahrens dient. Es braucht lediglich das Gefäß von der einen in die andere Mulde umgestellt zu werden, wofür wenig Zelt benötigt wird.

Die Heizmulde kann einen beheizbaren, wandförmigen Aufsatz aufweisen, der einen oberen Gefäßbereich umgibt, wenn sich das Gefäß in der Heizmulde befindet. Auf diese Weise läßt sich der Erhitzungsvorgang noch gleichmäßiger durchführen, was zu einer weiteren und vorteilhaften Verringerung von Temperaturgradienten in der Flüssigkeit bzw. im Transplantat führt.

Die Temperiervorrichtung ist weiterhin mit einem Einstellschalter versehen, der eine Längenskala aufweist, sowie mit einer Steuereinrichtung, die in Übereinstimmung mit der Stellung des Einstellschalters die zweite Zeitspanne einstellt.

Wie bereits erwähnt, kann z. B. bei schichtförmigen Transplantaten die Schichtdicke über den Einstellschalter eingegeben werden, woraufhin dann die Steuereinrichtung die zu dieser Schichtdicke gehörende zweite Zeitspanne auswählt, die z. B. in einer innerhalb des Gerätes vorhandenen Speichereinrichtung gespeichert sein kann. Nach Einstellung des Einstellschalters und Drücken einer Starttaste des Geräts läuft dann der Heizvorgang automatisch ab, der die genannte erste Zeitspanne und eine entsprechend ausgewählte zweite Zeitspanne umfaßt.

Im Bereich der Kühlmulde kann das Gerät mit Kühlschlitzen versehen sein, um mit Hilfe einer im Gerät vorhandenen Ventilatoreinrichtung Kühlluft in die Kühlmulde einblasen zu können.

Das Gerät kann ferner eine Tastatur zur Eingabe von weiteren Daten sowie einen Drucker zur Datenausgabe enthalten. Diese Daten lassen sich z. B. zur Kennzeichnung des Gefäßes und des Auffangbehälters verwenden, wozu der Drucker entsprechende Etiketten bedrucken und ausgeben kann.

Das bereits genannte Gefäß zur Aufnahme des Knochentransplantats besteht vorzugsweise aus Glas und weist an seinem oberen Rand ein Außengewinde auf, auf das ein Deckel aufgeschraubt werden kann. Zwischen dem stirnseitigen Rand des Gefäßes und dem Deckel befindet sich vorzugsweise ein Dichtungsring, um das Innere des Gefäßes hermetisch abdichten zu können.

Der genannte Deckel weist eine zentrale Öffnung auf, in die ein selbstabdichtender Gummipfropfen dicht eingesetzt ist. Durch diesen Gummipfropfen kann später ein Transfer-Set hindurchgestoßen werden, um die im Gefäß vorhandene Flüssigkeit ableiten zu können. Dieses Transfer-Set besteht aus zwei parallelen und in Axialrichtung gegeneinander versetzten Kanülen, von denen eine zur Ableitung von Flüssigkeit und die andere zur Belüftung dient.

Die zentrale Öffnung des Gefäßdeckels ist vorzugsweise durch eine sich axial nach außen erstreckende Zylinderwand fortgesetzt, auf die eine Abdeckung aufschraubbar ist. Durch diese Abdeckung läßt sich der Gummipfropfen in der zentralen Öffnung sichern. Die Zylinderwand und der Deckel sind vorzugsweise einstückig miteinander verbunden.

Nach einer anderen Ausgestaltung der Erfindung ist die zentrale Öffnung durch eine sich axial nach innen erstreckende Zylinderwand fortgesetzt, die ein den Gummipfropfen durchstoßendes Transfer-Set in Axialrichtung überragt.

Soll die Flüssigkeit aus dem Gefäß abgleitet werden, und zwar mit Hilfe des Transfer-Sets, so wird das Gefäß so gedreht, daß der Deckel nach unten weist. In diesem Fall kommt das Knochentransplantat auf der Innenseite des Gefäßdeckels zu liegen und könnte ohne die sich nach innen erstreckende Zylinderwand durch das Transfer-Set beschädigt werden. Andererseits wäre auch eine Beschädigung des Transfer-Sets durch das Knochentransplantat möglich, was jedoch durch die Zylinderwand verhindert wird, die die ins Innere des Gefäßes ragende Spitze des Transfer-Sets zu diesem Zweck hinreichend überragt. Mit anderen Worten dient diese nach innen weisende Zylinderwand sowohl zum Schutz des Knochentransplantats als auch zum Schutz des Transfer-Sets.

Vorzugsweise weist die sich axial nach innen erstreckende Zylinderwand benachbart zur Deckelinnenfläche liegende Radialschlitze auf, die eine solche Weite besitzen, daß eine gewissen Filterwirkung beim Ablassen der Flüssigkeit aus dem Gefäß erzielt wird. Evtl. Rückstände verbleiben dann zwischen dem äußeren Umfangsbereich der sich nach innen erstreckenden Zylinderwand und dem inneren Umfangsbereich des Gefäßes. Diese Rückstände fallen auf den Gefäßboden zurück, wenn es wieder mit dem Deckel nach oben positioniert wird.

Nach einer sehr vorteilhaften weiteren Ausgestaltung der Erfindung befindet sich oberhalb des Bodens des Gefäßes ein parallel zu ihm liegendes Gitter, auf das das Knochentransplantat beim Einführen in das Gefäß aufgelegt wird, wobei sich im Bereich zwischen dem Gitter und dem Gefäßboden die bereits erwähnten Magnetteilchen befinden, die mit Hilfe des im Gerät vorhandenen Magnetdrehwerks antreibbar sind, um für eine Verwirbelung der Flüssigkeit zu sorgen. Der Abstand des Gitters oberhalb des Gefäßbodens ist so gewählt, daß noch hinreichend Platz für die Aufnahme des Knochentransplantats zur Verfügung steht. Das Gitter ist fest mit den Seitenwänden des Gefäßes verbunden.

Die Erfindung wird nachfolgend unter Bezugnahme auf die Zeichnung näher beschrieben. Es zeigen:
**Fig. 1** eine Vorderansicht eines Ausführungsbeispiels einer Temperiervorrichtung nach der Erfindung,
**Fig. 2** eine Seitenansicht der Temperiervorrichtung nach Fig. 1 im Schnitt,
**Fig. 3** eine Draufsicht der Temperiervorrichtung nach Fig. 1, und
**Fig. 4** ein mit einem Auffangbehälter über ein Transfer-Set verbundenes Gefäß mit darin vorhandenem Knochentransplantat.

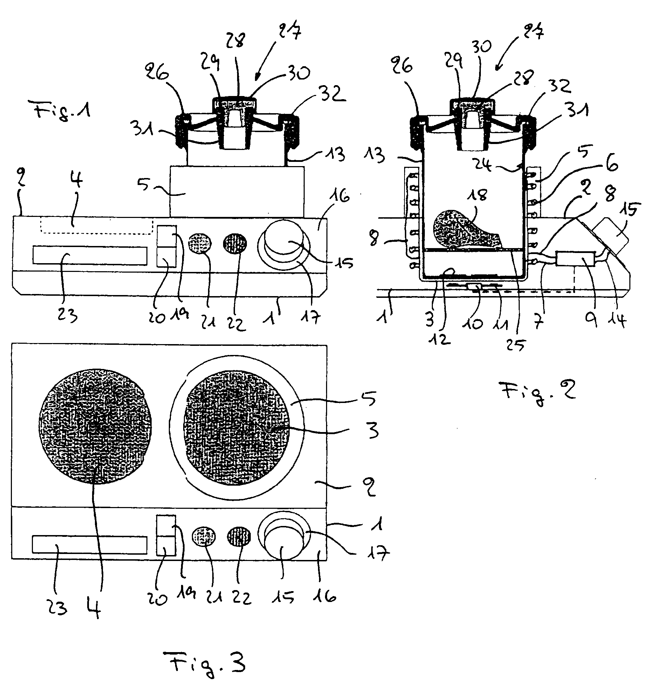

Die Figuren 1 bis 3 stellen jeweils verschiedene Ansichten einer Temperiereinrichtung zur Durchführung des erfindungsgemäßen Verfahrens dar.

Diese Temperiereinrichtung enthält ein Gehäuse 1, das eine horizontal liegende Oberfläche 2 aufweist, in die eine Heizmulde 3 und eine Kühlmulde 4 eingebracht sind. Beide Mulden 3 und 4 sind im Querschnitt kreisförmig ausgebildet und weisen denselben Innendurchmesser auf. Auf der Oberfläche 2 befinden sich entlang des Rands der Heizmulde 3 ein beheizbarer, wandförmiger Aufsatz 5, der z. B. eine Widerstandsheizung in Form eines wendelförmig geformten Heizdrahts 6 aufnimmt. Dieser Heizdraht 6 umgibt auch die Wand der Heizmulde 3 und erstreckt sich bis in deren unteren Bereich. Der wandförmige Aufsatz 5 kann beispielsweise aus Aluminium hergestellt sein.

Der Widerstandsdraht 6 ist über Stromleitungen 7 und 8 mit einer Steuereinrichtung 9 verbunden, die während einer ersten und in einer sich daran anschließenden zweiten Zeitspanne einen Strom durch den Heizdraht 6 hindurchschickt, um auf diese Weise die Heizmulde 3 sowie den wandförmigen Aufsatz 5 beheizen zu können.

Desweiteren ist mit der Steuereinrichtung 9 ein Magnetdrehwerk 10 verbunden, das sich unterhalb des Bodens der Heizmulde 3 befindet. Ein derartiges Magnetdrehwerk 10 kann auch unterhalb des Bodens der Kühlmulde 4 vorhanden sein, obwohl dies nicht explizit dargestellt ist. Wird das Magnetdrehwerk 10 gedreht, so rotieren mit ihm verbundene Magnete 11, die ihrerseits Magnete 12 mitnehmen, welche sich am Boden eines Gefäßes 13 befinden, das in die Heizmulde 3 eingesetzt ist. Im Gefäß 13 befindliche Flüssigkeit wird auf diese Weise durch die im Gefäß befindlichen Magnete 12 verwirbelt. Dadurch läßt sich bei der Beheizung der im Gefäß 13 vorhandenen Flüssigkeit ein besserer Temperaturausgleich erzielen.

Das Magnetdrehwerk 10 kann über die Steuereinrichtung 9 entweder elektrisch oder mechanisch angetrieben werden.

Die Steuereinrichtung 9 ist ferner über Leitungen 14 mit einem Einstellschalter 15 verbunden, der sich an einer Bedienungsfläche 16 des Gehäuses 1 befindet. Über diesen Einstellschalter 15, der z. B. als Drehschalter ausgebildet ist, läßt sich eine geometrische Abmessung des Knochentransplantats eingeben, auf deren Grundlage die Dauer der zweiten Zeitspanne durch die Steuereinrichtung 9 festgelegt wird. Hierzu ist der Einstellschalter 15 von einer Skala 17 umgeben, die eine Längenskala ist. Ist das Knochentransplantat, das in Fig. 2 mit dem Bezugszeichen 18 versehen ist, z. B. scheibenförmig ausgebildet, so wird mit Hilfe des Einstellschalters 15 die Dicke der Scheibe eingegeben, die zuvor bestimmt worden ist. In Abhängigkeit der eingegebenen Dicke wird dann aus einem in der Steuereinrichtung 9 vorhandenen Speicher (Tabelle) die zugehörige Dauer der zweiten Zeitspanne ausgelesen, wobei die Beziehungen zwischen den jeweiligen Dicken und den dazugehörigen zweiten Zeitspannen zuvor empirisch ermittelt worden sind.

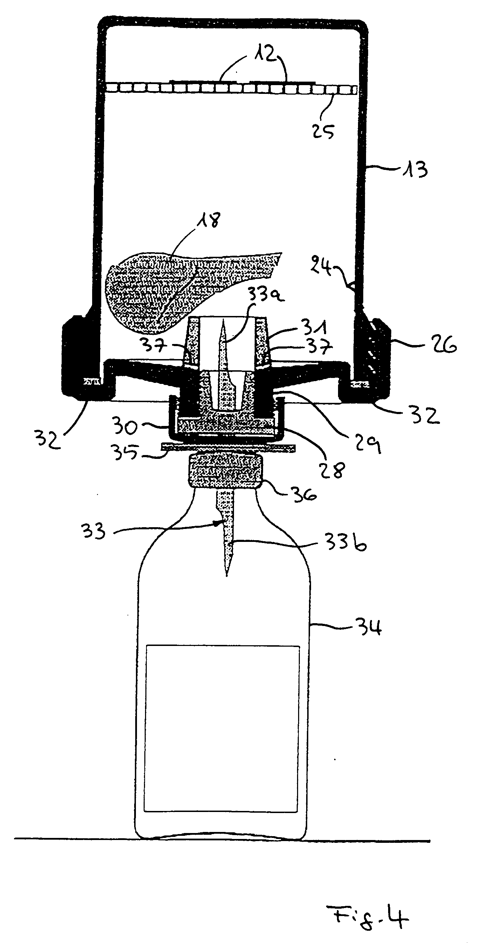

Auf der Bedienungsfläche befinden sich ferner ein Startschalter 19 zum Starten des Heizbetriebs sowie ein Startschalter 20 zum Starten des Kühlbetriebs. Ihnen sind entsprechende Anzeigelampen 21 und 22 zugeordnet. Ein Drucker 23 dient u. a. zum Ausdrucken von Etiketten, die sich zur Kennzeichnung des Gefäßes 13 sowie eines in Fig. 4 dargestellten Auffangbehälters verwenden lassen. Diese Etiketten werden mit Daten bzw. Kennungsdaten bedruckt, die über eine nicht dargestellte Tastatur in das Gerät eingegeben werden können.

Die Kühlmulde 4 kann über nicht gezeigte Schlitze mit Kühlluft versorgt werden, die aus einem ebenfalls nicht dargestellten Aggregat im Innern des Geräts 1 abgegeben wird.

Wie in Fig. 2 zu erkennen ist, weist das Gefäß 13 eine solche Größe auf, daß es passend in die Heizmulde 3 eingesetzt werden kann. Dabei erstreckt sich der wandförmige Aufsatz bis nahe zum oberen Rand des Gefäßes 13 bzw. bis zu einer Markierung 24 an der Wandfläche des Gefäßes 13, bis zu der das Gefäß 13 mit Flüssigkeit aufgefüllt wird. Die Flüssigkeitsoberfläche fluchtet daher mit dem oberen Rand des wandförmigen Aufsatzes 5, so daß sich die Flüssigkeit schnell aufheizen läßt. Die Markierung 24 kann auch unterhalb des oberen Rands des Aufsatzes 5 liegen, wenn kleinere Transplantate 18 zu desinfizieren sind, jedoch muß in jedem Fall dafür gesorgt sein, daß das Transplantat 18 vollständig mit Flüssigkeit bedeckt ist.

Das Transplantat 18 liegt im Innern des Gefäßes 13 auf einem Gitter 25, das in einem gewissen Abstand vom Boden des Gefäßes 13 parallel zu diesem fest angeordnet ist. Zwischen Boden und Gitter befinden sich die Magnetteilchen 12, die zur Verwirbelung der Flüssigkeit im Gefäß 13 dienen. Darüber hinaus kann sich im Bereich zwischen dem Gitter 25 und dem Boden des Gefäßes 13 Restflüssigkeit ansammeln, nachdem die im Gefäß 13 vorhandene Flüssigkeit im wesentlichen abgelassen worden ist, wie noch zu beschreiben ist, so daß sich das Transplantat 18 im Gefäß 13, ohne von Flüssigkeit umgeben zu sein, quasi trocken einfrieren läßt.

Die Gefäßöffnung ist mit einem Deckel 26 verschlossen, der auf ein Gewinde am oberen Außenrand des Gefäßes 13 aufgeschraubt wird. Dieser Deckel 26 besteht aus Kunststoffmaterial und kann nach geeigneter Sterilisation mehrmals verwendet werden.

Der Deckel 26 weist eine zentrale Öffnung 27 auf, in die ein selbstabdichtender Gummipfropfen 28 dicht eingesetzt ist. Ferner ist die zentrale Öffnung 27 durch eine sich axial nach außen erstreckende Zylinderwand 29 fortgesetzt, auf die eine weitere Abdeckung 30 aufgeschraubt ist. Diese weitere Abdeckung 30 dient quasi zum Schutz des Gummipfropfens 28. Darüber hinaus ist die zentrale Öffnung 27 durch eine sich axial nach innen erstreckende Zylinderwand 31 fortgesetzt, die also ins Innere des Gefäßes 13 hineinragt. Der Deckel 26 sowie die Zylinderwände 29 und 31 können z. B. einstückig ausgebildet sein.

Es sei noch darauf hingewiesen, daß zwischen dem stirnseitigen Rand des Gefäßes 13 und dem Deckel 26 ein Dichtungsring 32 liegen kann, um für einen hermetischen Abschluß des Gefäßes 13 zu sorgen.

Die Fig. 4 zeigt die Lage des Gefäßes 13 für den Fall, daß die in ihm vorhandene Flüssigkeit abgelassen werden soll. Dabei wird die Flüssigkeit mit Hilfe eines Transfer-Sets 33 in einen Auffangbehälter 34 geleitet, der beispielsweise eine sterilisierte und ggf. mit einer Nährlösung gefüllte Flasche sein kann.

Das Transfer-Set 33 besteht aus zwei gleichlangen Kanülen 33a, 33b, die parallel zueinander liegen und in Längsrichtung gegeneinander versetzt sind. Eine Querverstrebung 35 dient als Anschlag, um den Einschub des Transfer-Sets 33 in das Gefäß 13 bzw. in den Auffangbehälter 34 zu begrenzen.

Gemäß Fig. 4 durchragt das Transfer-Set einerseits sowohl die Abdeckung 30 als auch den Gummipfropfen 28 des Gefäßes 13 und andererseits einen Deckel 36 des Auffangsbehälters 34. Sowohl der Gummipfropfen 28 als auch der Deckel 36 bestehen aus einem selbstabdichtenden Material, so daß sich die durch das Eindringen des Transfer-Sets 33 verursachte Öffnung in diesem Material von selbst wieder schließt, wenn das Transfer-Set 33 wieder herausgenommen wird.

Entsprechend der Darstellung von Fig. 4 überragt die Spitze der Kanüle 33a des Transfer-Sets 33 die Querversteifung 35 nur soweit, daß sie nach Einsetzen in das Gefäß 13 den freien Rand des Zylinders 31 nicht überragt, wodurch das Transplantat 18 gegen Beschädigungen durch die Spitze der Kanüle 33a geschützt wird, und umgekehrt. Die Querverstrebung 35 dient hier praktisch als Anschlag. Sie ist einstückig mit den Kanülen 33a und 33b verbunden.

Wie weiter zu erkennen ist, befinden sich Radialschlitze 37 in der Seitenwand des nach innen gerichteten Zylinders 31, und zwar in einem Bereich nahe der Innenseite des Deckels 26. Diese Radialschlitze 37 dienen als Filterschlitze, um beim Ableiten von Flüssigkeit aus dem Gefäß 13 Rückstände zurückzuhalten. Zu diesem Zweck kann das Gefäß 13 nach Einsetzen des Transfer-Sets 33 zunächst leicht geneigt werden, um die Flüssigkeit durch die Radialschlitze 37 hindurch ablaufen zu lassen. Die Ableitung würde in diesem Fall über die Kanüle 33b erfolgen, während die Kanüle 33a zur Belüftung dient. Rückstände würden sich dann im Bereich zwischen der Außenwand des Zylinders 31 und der Innenwand des Gefäßes 13 ansammeln.

Nach Entleerung des Gefäßes 13 und Herausnahme des Transfer-Sets 33 aus dem Deckel 26 wird das Gefäß 13 mit seinem Boden auf eine Unterlage gestellt, so daß das Knochentransplantat 18 schließlich auf dem Gitter 25 zu liegen kommt. Eine evtl. im Gefäß 13 noch vorhandene Restflüssigkeit sowie die erwähnten Rückstände würden sich dann im Bereich zwischen dem Gitter 25 und dem Boden des Gefäßes 13 ansammeln, so daß eine trockene Lagerung des Knochentransplantats 18 auf dem Gitter 25 möglich ist. In diesem Zustand wird das nach wie vor verschlossene Gefäß 13 in eine Kühlkammer gestellt, um das Knochentransplantat 18 einzufrieren. Die im Auffangbehälter 34 vorhandene Flüssigkeit wird labormäßig untersucht, um das Ergebnis des Desinfektionsverfahrens zu überprüfen.

## Patentansprüche

1. Verfahren zum Desinfizieren von Knochentransplantaten, insbesondere von humanen Spongiosa-Transplantaten, mit folgenden Schritten:
a) Bereitstellen und Ausmessen der Größe des Knochentransplantats (18),
b) Einlegen des Knochentransplantats (18) in ein Gefäß (13) mit vorbestimmtem Fassungsvermögen,
c) Auffüllen des Gefäßes (13) mit einer sterilen Flüssigkeit bis zu einer für alle Knochentransplantatgrößen vorgesehenen Gefäßmarkierung (24) oberhalb des Knochentransplantats (18),
d) Verschließen des Gefäßes (13) mit einem einen selbstabdichtenden Durchstoßbereich (28) aufweisenden Deckel (26),
e) Einsetzen des verschlossenen Gefäßes (13) in ein Heizgerät (1) und Erhitzen des Gefäßes (13) über eine konstante erste Zeitspanne auf eine Endtemperatur sowie Halten dieser Endtemperatur über eine in fester Beziehung zu der ausgemessenen Größe des Knochentransplantats (18) stehenden zweiten Zeitspanne,
f) Abkühlen des Gefäßes (13) auf Raumtemperatur,
g) Durchstoßen des Durchstoßbereichs (28) des Deckels (26) sowie eines Durchstoßbereichs eines anderen Deckels (36) eines Auffangbehälters (34) mittels eines Transfer-Sets (33) zur Ableitung der Flüssigkeit aus dem Gefäß (13) in den Auffangbehälter (34), und
h) Herausnehmen des Transfer-Sets (33) aus dem Gefäßdeckel sowie Einbringen des Gefäßes (13) in einen Kühlraum zwecks Einfrieren des Knochentransplantats (18).

2. Verfahren nach Anspruch 1, **dadurch gekennzeichnet**, daß die Flüssigkeit im Gefäß (13) während der ersten Zeitspanne auf eine Temperatur von etwa 80°C erhitzt wird.

3. Verfahren nach Anspruch 1 oder 2, **dadurch gekennzeichnet**, daß beim Erhitzen und Abkühlen eine Verwirbelung der im Gefäß (13) vorhandenen Flüssigkeit erfolgt.

4. Verfahren nach Anspruch 1, 2 oder 3, **dadurch gekennzeichnet**, daß die Flüssigkeit beim Umfüllen vom Gefäß (13) in den Auffangbehälter (34) filtriert wird, um Rückstände im Gefäß (13) zurückzuhalten.

5. Verfahren nach einem der Ansprüche 1 bis 4, **dadurch gekennzeichnet**, daß das Gefäß (13) nach Ableitung der Flüssigkeit so positioniert wird, daß das Knochentransplantat (18) getrennt von einer Restflüssigkeit auf einem im Gefäßinnern vorhandenen Gitter (25) zu liegen kommt.

6. Verfahren nach einem der Ansprüche 1 bis 5, **dadurch gekennzeichnet,** daß als Flüssigkeit steriles Wasser verwendet wird.

7. Einrichtung zur Durchführung des Verfahrens nach einem der Ansprüche 1 bis 6, **gekennzeichnet durch:**
- eine Temperiervorrichtung (1) mit einer Heizmulde (3) und einer Kühlmulde (4);
- ein Gefäß (13) zum Einlegen des Knochentransplantats (18) mit vorbestimmtem Fassungsvermögen und einen einen selbstabdichtenden Durchstoßbereich aufweisenden Deckel (26);
- einen Auffangbehälter (34) mit einem einen selbstabdichtenden Durchstoßbereich aufweisenden Deckel (36);
- ein Transfer-Set (33) zur Ableitung der sterilen Flüssigkeit aus dem Gefäß (13) in den Auffangbehälter (34); und
- eine Steuereinrichtung zum Steuern des Erhitzens bzw. des Abkühlens des Gefäßes (13).

8. Einrichtung nach Anspruch 7, **dadurch gekennzeichnet,** daß die Heizmulde (3) einen beheizbaren, wandförmigen Aufsatz (5) aufweist, der einen oberen Gefäßbereich umgibt, wenn sich das Gefäß (13) in der Heizmulde (3) befindet.

9. Einrichtung nach Anspruch 7 oder 8, **dadurch gekennzeichnet,** daß sie einen mit einer Längenskala versehenen Einstellschalter (15) aufweist und die Steuereinrichtung (9) in Übereinstimmung mit der Stellung des Einstellschalters (15) die zweite Zeitspanne einstellt.

10. Einrichtung nach einem der Ansprüche 7 bis 9, **dadurch gekennzeichnet,** daß sie ein Magnetdrehwerk (10) enthält, um im Innern des Gefäßes (13) befindliche Magnetteilchen (12) anzutreiben.

11. Einrichtung nach einem der Ansprüche 7 bis 10, **dadurch gekennzeichnet,** daß sie eine Tastatur zur Eingabe von Daten und einen Drucker (23) zur Datenausgabe enthält.

12. Einrichtung nach einem der Ansprüche 7 bis 11, **dadurch gekennzeichnet,** daß der Deckel (26) auf das Gefäß (13) aufschraubbar und zwischen Deckel und stirnseitigem Gefäßrand ein Dichtungsring (32) angeordnet ist.

13. Einrichtung nach Anspruch 12, **dadurch gekennzeichnet,** daß im Deckel (26) eine zentrale Öffnung (27) vorhanden ist, in die ein selbstabdichtender Gummipfropfen (28) dicht eingesetzt ist.

14. Einrichtung nach Anspruch 13, **dadurch gekennzeichnet,** daß die zentrale Öffnung (27) durch eine sich axial nach außen erstreckende Zylinderwand (29) fortgesetzt ist, auf die eine Abdeckung (30) aufschraubbar ist.

15. Einrichtung nach Anspruch 13 oder 14, **dadurch gekennzeichnet,** daß die zentrale Öffnung (27) durch eine sich axial nach innen erstreckende Zylinderwand (31) fortgesetzt ist, die des den Gummipfropfen (28) durchstoßende Transfer-Set (33) in Axialrichtung überragt.

16. Einrichtung nach Anspruch 15, **dadurch gekennzeichnet,** daß die sich axial nach innen erstreckende Zylinderwand (31) benachbart zur Deckelinnenfläche liegende Radialschlitze (37) aufweist.

17. Einrichtung nach einem der Ansprüche 7 bis 16, **dadurch gekennzeichnet,** daß oberhalb des Bodens des Gefäßes (13) ein parallel zu ihm liegendes Gitter (25) angeordnet ist, auf das das Knochentransplantat (18) beim Einführen in das Gefäß (13) aufgelegt wird, und daß sich die Magnetteilchen (12) im Bereich zwischen dem Gitter (25) und dem Gefäßboden befinden.

## Claims

1. Method of sterilizing bone grafts, especially human spongiosa grafts, comprising the following steps:
a) providing the bone graft (18) and evaluating its size,
b) placing the bone graft (18) in a vessel (13) of predetermined capacity,
c) making up the vessel (13) with a sterile liquid to a vessel marking (24), above the bone graft (18), which is provided for all sizes of bone graft,
d) sealing the vessel (13) with a lid (26) having a self-sealing penetration region (28),
e) inserting the sealed vessel (13) into a heating apparatus (1) and heating the vessel (13) for a constant first period of time to a final temperature and maintaining this final temperature for a second period of time which is fixedly related to the measured size of the bone graft (18),
f) cooling the vessel (13) to room temperature,
g) penetrating the penetration area (28) of the lid (26) and a penetration area of another lid (36) of a collecting container (34), by means of a transfer set (33) for transferring the liquid from the vessel (13) into the collecting container (34), and
h) removing the transfer set (33) from the vessel lid and introducing the vessel (13) into a cooling chamber in order to freeze the bone graft (18).

2. Method according to Claim 1, characterized in that the liquid in the vessel (13) is heated during the first period of time at a temperature of about 80°C.

3. Method according to Claim 1 or 2, characterized in that, as it is heated and cooled, the liquid present in the vessel (13) is fluidized.

4. Method according to Claim 1, 2 or 3, characterized in that as it is transferred from the vessel (13) to the collecting container (34) the liquid is filtered in order to retain residues in the vessel (13).

5. Method according to one of Claims 1 to 4, characterized in that following transfer of the liquid the vessel (13) is Positioned so that the bone graft (18) comes to lie, separated from any residual liquid, on a grid (25) present in the interior of the vessel.

6. Method according to one of Claims 1 to 5, characterized in that sterile water is used as the liquid.

7. Apparatus for implementing the method according to one of Claims 1 to 6, characterized by:
- a thermal conditioning device (1) having a heating pan (3) and a cooling pan (4);
- a vessel (13) for insertion of the bone graft (18), of predetermined capacity, and a lid (26) which has a self-sealing penetration area;
- a collecting container (34) with a lid (36) having a self-sealing penetration region;
- a transfer set (33) for conveying the sterile liquid from the vessel (13) into the collecting container (34); and
- a control device for controlling the heating and cooling, respectively, of the vessel (13).

8. Apparatus according to Claim 7, characterized in that the heating pan (33) has a heatable, wall-like top section (5) which surrounds an upper region of the vessel when the vessel (13) is in the heating pan (3).

9. Apparatus according to Claim 7 or 8, characterized in that it has a setting switch (15) provided with a length scale and the control device (9) in agreement with the setting of the setting switch (15) sets the second period of time.

10. Apparatus according to one of Claims 7 to 9, characterized in that it comprises a magnetic rotator (10) in order to drive magnetic particles (12) located in the interior of the vessel (13).

11. Apparatus according to one of Claims 7 to 10, characterized in that it comprises a keyboard for data input and a printer (23) for data output.

12. Apparatus according to one of Claims 7 to 11, characterized in that the lid (26) can be screwed onto the vessel (13), and there is a sealing ring (32) between lid and end-face vessel edge.

13. Apparatus according to Claim 12, characterized in that in the lid (26) there is a central aperture (27) into which a self-sealing rubber plug (28) is inserted tightly.

14. Apparatus according to Claim 13, characterized in that the central aperture (27) is continued by an axially outwardly extending cylinder wall (29) onto which a cover (30) can be screwed.

15. Apparatus according to Claim 13 or 14, characterized in that the central aperture (27) is continued by an axially inwardly extending cylinder wall (31) which projects axially beyond the transfer set (33) which penetrates the rubber plug (28).

16. Apparatus according to Claim 15, characterized in that the axially inwardly extending cylinder wall (31) has radial slots (37) adjacent to the inner surface of the lid.

17. Apparatus according to one of Claims 7 to 16, characterized in that above the base of the vessel (13) and lying parallel with it is a grid (25) onto which the bone graft (18) is placed on its introduction into the vessel (13), and in that the magnetic particles (12) are located within the area between the grid (25) and the vessel base.

## Revendications

1. Procédé de désinfection de transplants osseux, en particulier de transplants de tissus osseux spongieux humains, comportant les étapes ci-après :
(a) préparation et dimensionnement de la taille du transplant osseux (18),
(b) insertion du transplant osseux (18) dans un récipient (13) ayant une contenance prédéterminée,
(c) remplissage du récipient (13) avec un liquide stérile, jusqu'à un repère du récipient (24) prévu pour toutes les tailles de transplant osseux, à un niveau supérieur à celui du transplant osseux (18),
(d) obturation du récipient (13) avec un couvercle (26) présentant une zone de percement (28) auto-scellable,
(e) insertion du récipient (13) fermé dans un appareil de chauffage (1) et chauffage du récipient (13) sur un premier intervalle de temps constant, à une température finale, ainsi que maintien de cette température finale sur un deuxième intervalle de temps choisi en fonction de la taille mesurée du transplant osseux (18),
(f) refroidissement du récipient (13) à la température ambiante,
(g) transpercement de la zone de percement (28) du couvercle (26) [ainsi qu'une zone de percement d'un autre couvercle] (36) d'un récipient récepteur (34), au moyen d'un jeu d'appareillage de transfert (3) destiné à déplacer du liquide du récipient (13) dans le récipient récepteur (34), et
(h) extraction du jeu d'appareillage de transfert (33) hors du couvercle de récipient, ainsi qu'insertion du récipient (13) dans une enceinte de refroidissement, dans le but de congeler le transplant osseux (18).

2. Procédé selon la revendication 1, caractérisé en ce que le liquide se trouvant dans le récipient (13) est chauffé à une température d'environ 80°C pendant le premier intervalle de temps.

3. Procédé selon la revendication 1 ou 2, caractérisé en ce que, lors du chauffage et du refroidissement, est effectuée une mise en tourbillon du liquide se trouvant dans le récipient (13).

4. Procédé selon la revendication 1, 2 ou 3, caractérisé en ce que le liquide est filtré lors du transvasement du récipient (13) dans le récipient récepteur (34), afin de retenir les résidus dans le récipient (13).

5. Procédé selon l'une quelconque des revendications 1 à 4, caractérisé en ce que le récipient (13) après soutirage du liquide est positionné de manière que le transplant osseux (18) après avoir été séparé du liquide résiduel, vienne se placer sur une grille (25) disposée à l'intérieur du récipient.

6. Procédé selon l'une des revendications 1 à 5, caractérisé en ce que l'on utilise de l'eau comme liquide stérile.

7. Dispositif de mise en oeuvre du procédé selon l'une quelconque des revendications 1 à 6, caractérisé en ce qu'il comporte :
- un dispositif de régulation de température (1) doté d'une cuve de chauffage (3) et d'une cuve de refroidissement (4);
- un récipient (13) destiné à recevoir le transplant osseux (18), d'une contenance prédéterminée et avec un couvercle (26) présentant une zone de percement auto-scellable;
- un récipient récepteur (34) avec un capuchon (36) présentant une zone de percement auto-scellable,
- un jeu d'appareillage de transfert (33) pour transférer le liquide stérile du récipient (13) dans le récipient récepteur (34); et
- un dispositif de régulation destiné à réguler le chauffage et/ou le refroidissement du récipient (13).

8. Un dispositif selon la revendication 7, caractérisé en ce que la cuve de chauffage (3) présente un élément rapporté (5) en forme de paroi, chauffable, entourant une zone supérieure de récipient lorsque ce récipient (13) se trouve dans la cuve de chauffage (3).

9. Un dispositif selon la revendication 7 ou 8, caractérisé en ce qu'il présente une commande de réglage (15) dotée d'une échelle de longueur et le dispositif de commande (9) règle le deuxième intervalle de temps en fonction de la position de la commande de réglage (15).

10. Un dispositif selon l'une des revendications 7 à 9, caractérisé en ce qu'il contient un organe rotatif magnétique (10), destiné à entraîner les particules métalliques (12) se trouvant à l'intérieur du récipient (13).

11. Un dispositif selon l'une quelconque des revendications 7 à 10, caractérisé en ce qu'il comporte un clavier pour introduire des données et une imprimante (23) pour éditer des données.

12. Un dispositif selon l'une quelconque des revendications 7 à 11, caractérisé en ce que le couvercle (26) est installé sur le récipient (13) par un montage par vissage et, entre le couvercle et le bord frontal du récipient, est disposée une bague d'étanchéité (32).

13. Un dispositif selon la revendication 12, caractérisé en ce que dans le couvercle (26) est disposée une ouverture centrale (27) dans laquelle un bouchon en caoutchouc (28) auto-scellable est inséré de façon étanche.

14. Un dispositif selon la revendication 13, caractérisé en ce que l'ouverture centrale (27) est prolongée par une paroi cylindrique (29) s'étendant axialement vers l'extérieur, sur laquelle un capuchon (30) peut être monté par vissage.

15. Un dispositif selon la revendication 13 ou 14, caractérisé en ce que l'ouverture centrale (27) est prolongée par une paroi cylindrique (31) s'étendant axialement vers l'intérieur, qui dépasse en direction axiale du jeu d'appareillage de transfert (33) transperçant le bouchon en caoutchouc (28).

16. Un dispositif selon la revendication 15, caractérisé en ce que la paroi cylindrique (31) s'étendant axialement vers l'intérieur présente des fentes radiales (37) prévues au voisinage de la surface intérieure de couvercle.

17. Un dispositif selon l'une des revendications 7 à 16, caractérisé en ce qu'au-dessus du fond du récipient (13) est disposée une grille (25), placée parallèlement au fond et sur laquelle le transplant osseux (18) est posé lors de son introduction dans le récipient (13), et en ce que les particules métalliques (12) se trouvent dans la zone située entre la grille (25) et le fond de récipient.
